(19) Europäisches Patentamt
European Patent Office
Office européen des brevets

(11) Numéro de publication: **0 235 000 B1**

# (12) FASCICULE DE BREVET EUROPEEN

(45) Date de publication du fascicule du brevet: **14.11.90**

(51) Int. Cl.⁵: **C07C 255/32**, C07B 59/00, C07D 233/68, G01N 33/534

(21) Numéro de dépôt: **87400197.7**

(22) Date de dépôt: **29.01.87**

(54) **Pyréthrinoïdes marqués à l'iode, leur procédé de préparation et leur application aux dosages radioimmunologiques.**

(30) Priorité: **29.01.86 FR 8601220**

(43) Date de publication de la demande: **02.09.87 Bulletin 87/36**

(45) Mention de la délivrance du brevet: **14.11.90 Bulletin 90/46**

(84) Etats contractants désignés: **CH DE FR GB IT LI NL**

(56) Documents cités: **US-A- 4 136 159**

(73) Titulaire: **ROUSSEL-UCLAF, 35, boulevard des Invalides, F-75007 Paris(FR)**

(72) Inventeur: **Demoute, Jean-Pierre, 249bis, rue de Rosny, F-93100 Montreuil-Sous-Bois(FR)**
Inventeur: **Touyer, Gaëtan, 143, avenue Jean Jaurès, F-75019 Paris(FR)**
Inventeur: **Mouren, Michel, 15, rue de Mirbel, F-75005 Paris(FR)**

(74) Mandataire: **Tonnellier, Marie-José et al, Département des Brevets ROUSSEL UCLAF B.P. no 9, F-93230 Romainville(FR)**

EP 0 235 000 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés pyréthrinoïdes radioactifs marqués à l'iode, leur procédé de préparation et leur application aux dosages radioimmunologiques.

Plus particulièrement, elle a pour objet des dérivés de formule générale (I):

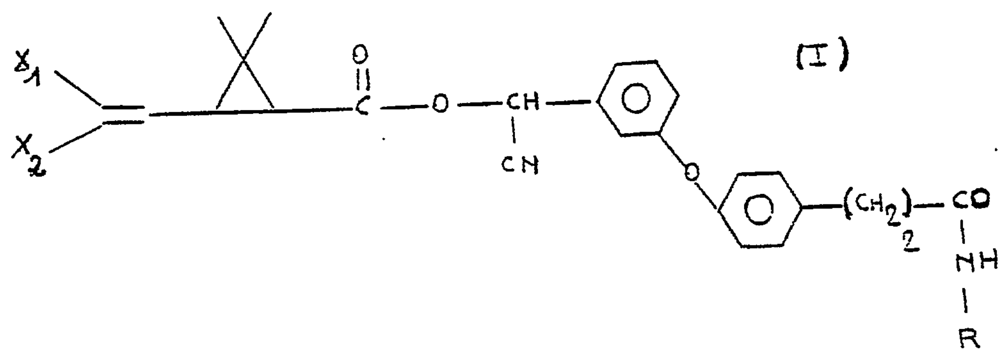

dans laquelle $X_1$ représente un atome d'halogène ou un radical trifluorométhyle, $X_2$ représente un atome d'halogène et R représente le reste d'un acide aminé $R{-}NH_2$ ou le reste d'un dérivé de ce dernier choisi dans le groupe constitué par l'histidine, la tryosine, l'histidinol, l'histamine, la tyramine et le tyrosinate de méthyle marqués à l'iode $^{125}$ ou $^{131}$, ces produits se présentant sous l'une quelconque de leurs formes isomères ou sous forme de mélanges d'isomères. $X_2$ et $X_1$ lorsqu'il représentent un atome d'halogène, peuvent être un atome de fluor, de chlore, de brome ou d'iode. Par mélange d'isomères, on entend tous les mélanges d'isomères possibles y compris les racémates.

L'invention a notamment pour objet les dérivés de formule (I) dans laquelle $X_1$ représente un atome de chlore ou de brome ou un radical trifluorométhyle et $X_2$ représente un atome de chlore ou de brome.

L'invention a plus particulièrement pour objet les dérivés de formule (I) dans laquelle $X_1$ et $X_2$ représentent un atome de brome et tout particulièrement:

le 1R-[1 alpha S, 2 alpha]4-[3-[cyano[[3-(2,2-dibromoéthényl) 2,2-diméthyl cyclopropyl]carbonyl]oxy]-méthyl]phénoxy]benzène-N-[2-(2-iodo $^{125}$I 4-1H-imidazolyl) éthyl]propanamide de formule:

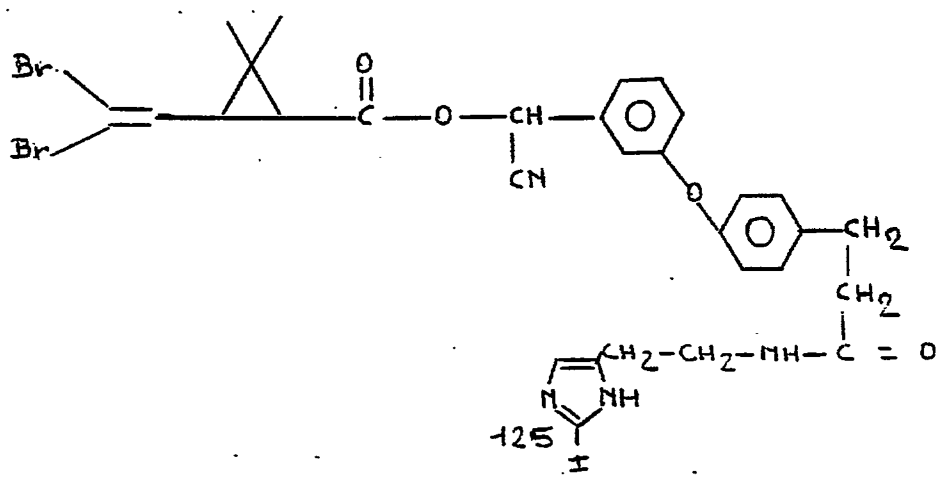

L'invention a également pour objet un procédé de préparation des produits répondant à la formule générale (I) ci-dessus.

Ce procédé est caractérisé en ce que l'on fait réagir un produit de formule (II):

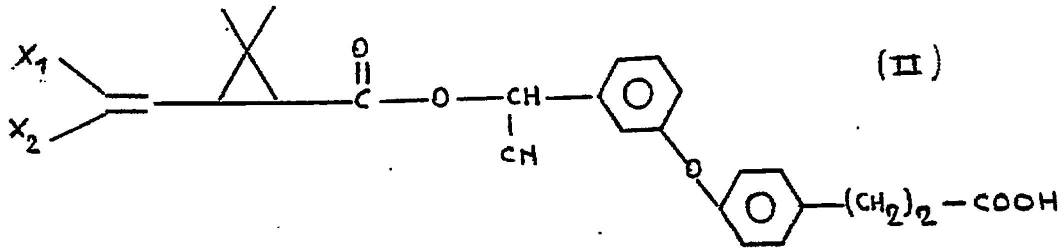

(II)

dans laquelle $X_1$ et $X_2$ ont la signification précitée, ce produit se présentant sous l'une quelconque de ses formes isomères ou sous forme de mélanges d'isomères, avec un halogénoformiate d'alcoyle dans lequel le radical alcoyle $R_1$ renferme au plus 6 atomes de carbone pour obtenir le produit de formule (III):

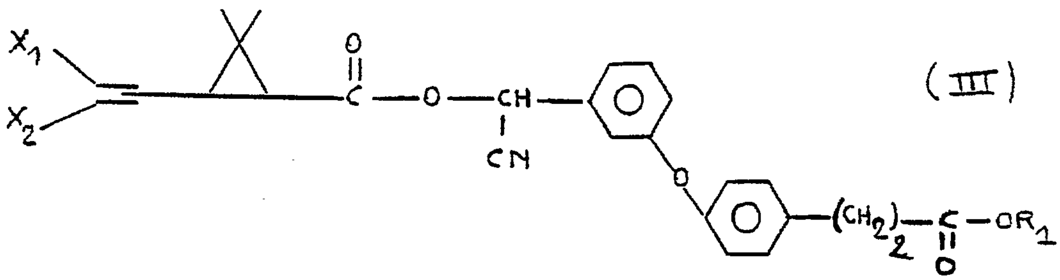

(III)

dans laquelle $X_1$ et $X_2$ ont les significations précitées que l'on fait réagir avec un acide aminé ou un dérivé d'acide aminé choisi dans le groupe constitué par l'histidine, la tyrosine, l'histidinol, l'histamine, la tyramine et tyrosinate de méthyle marqués à l'iode $^{125}$ ou $^{131}$ et obtient le produit cherché de formule générale (I) que l'on purifie par des moyens physiques.

Dans des conditions préférentielles de mise en œuvre du procédé:

– on fixe sur la fonction acide un groupement activateur de la fonction carbonyle en faisant agir un halogénoformiate d'alcoyle dans lequel le radical alcoyle renferme au plus 6 atomes de carbone et on opère en présence d'une base tertiaire, en milieu anhydre et sous atmosphère inerte.

Dans des conditions préférentielles de mise en œuvre, le procédé est caractérisé en ce que l'halogénoformiate d'alcoyle est le chloroformiate d'isobutyle et on opère en présence de tri-n-butyl amine.

L'invention a notamment pour objet un procédé de préparation des composés de formule générale (I) caractérisé en ce que l'acide aminé ou son dérivé que l'on fait réagir avec le produit de formule générale (III) dans laquelle $R_1$ représente un groupement activateur de la fonction carbonyle, est l'histamine marquée à l'iode $^{125}$ ou $^{131}$ ou un dérivé et on opère sous atmosphère inerte.

L'invention a plus précisément pour objet un procédé de préparation tel que défini précédemment, caractérisé en ce que l'on obtient le produit de formule:

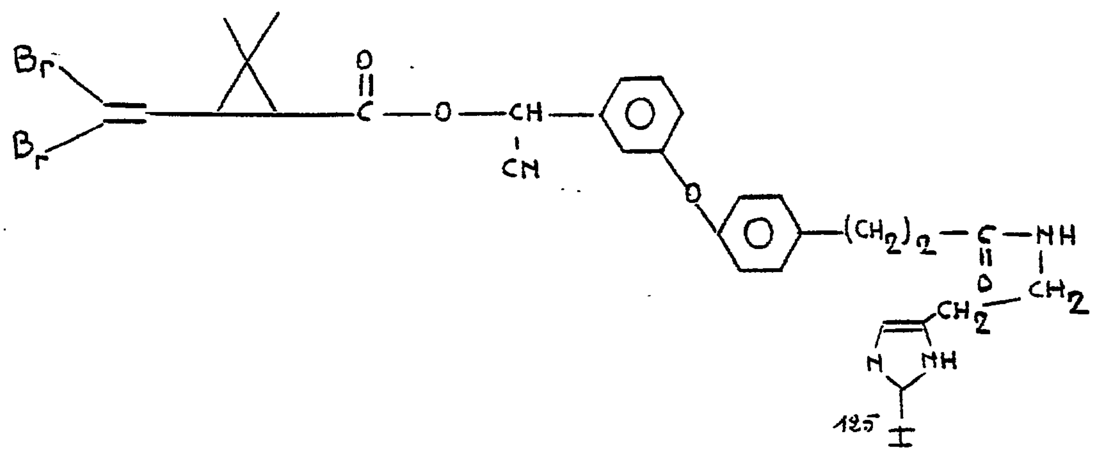

L'invention a également pour objet l'application des produits de formule générale (I) à l'étude et au dosage radioimmunologique des esters de l'alcool alpha-cyano 3-phénoxy benzylique correspondants (produit B) dans les fluides biologiques, chez l'homme ou l'animal.

L'invention a notamment pour objet l'application du 1R[1 alpha S,2alpha]4-[3-[cyano[[3-(2,2-dibromo éthényl) 2,2-diméthyl cyclopropyl]carbonyl] oxy]méthyl]phénoxy]benzène-N-[2-(2-iodo $^{125}I$ 4-1 H imidazolyl)éthyl]propanamide (produit A) à l'étude et aux dosages radioimmunologiques de la deltamethrine (produit $B_1$) dans les fluides biologiques, chez l'homme ou l'animal.

Les produits de formule générale I, objet de l'invention, peuvent être utilisés lors des études et lors du dosage radioimmunologique des produits B et notamment le produit A pour la deltaméthrine.

Lors de ces études, les produits de formule (I) permettent un dosage spécifique aisé de quantités de l'ordre de quelques dizaines de Picogrammes de produit B, sans que l'on soit obligé de recourir à des méthodes d'isolement et de purification par chromatographie, avant de procéder au dosage proprement dit selon des méthodes radioimmunologiques conventionnelles telles que celles décrites par:

- S.A. BERGSON et R.S. YALOW, HORMONE 4, p. 557 (1964) et
- G.E. ABRAHAM J. of CHEM. ENDOCRINAL METAB., 29, p. 866 (1969).

Les exemples suivants illustrent l'invention, sans toutefois la limiter.

Exemple 1: Acide 1R /1 alpha (R), 2 alpha /4-/3/cyano//(2-(2,2-dibromoéthényl) 3,3-diméthylcyclopropyl/ carbonyl oxy/méthyl/phénoxy/benzène propanoique.

Stade A: 4-/3-(1,3-dioxolan 2-yl) phénoxy/ benzaldéhyde.

On mélange 12,05 g de parahydroxybenzaldéhyde, 30 $cm^3$ de pyridine et 5,5 g de méthylate de sodium, chauffe pour distiller 4 $cm^3$ de pyridine, ajoute 2,5 g de chlorure cuivreux et 38 g de 2-(m-bromophényl) 1,3-dioxolane, chauffe en agitant énergiquement jusqu'à 200°C (température extérieure), tout en distillant la pyridine, et maintient à 200°C (température extérieur) pendant 5 heures. Après refroidissement, on reprend le résidu au chlorure de méthylène, lave par une solution aqueuse N d'acide chlorhydrique puis à l'eau. On sèche les phases organiques, concentre à sec par distillation sous pression réduite, chromatographie le résidu sur silice en éluant par un mélange hexane-acétate d'éthyle (6/4) et obtient 18,28 g de produit attendu.

Spectre IR (chloroforme):

2 830 $cm^{-1}$
2 738 $cm^{-1}$ aldéhyde

1 696 $cm^{-1}$ C = O

1 598 $cm^{-1}$
1 580 $cm^{-1}$ aromatique
1 500 $cm^{-1}$

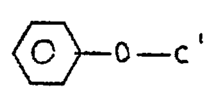
1 240 $cm^{-1}$

1 097 $cm^{-1}$
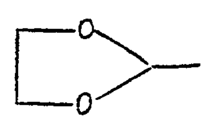
1 071 $cm^{-1}$
1 076 $cm^{-1}$

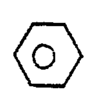
833 $cm^{-1}$

Spectre de RMN (CD $Cl_3$)

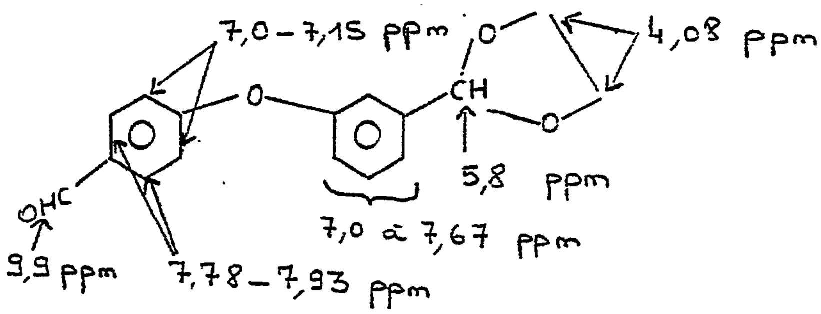


**Stade B** : 2-/3-(4-/delta E (1,1-diméthyléthoxy) 1-oxopropényl/phénoxy) phényl/1,3-dioxolane.

On mélange 137 cm3 de tétrahydrofuranne et 13,72 g de bromure de lithium, refroidit à -30°C, ajoute d'un coup 11,94 cm3 de diisopropylamine, puis un mélange de 10 g de 4-/3-(1,3-dioxolan 2-yl) phénoxy/ benzaldéhyde, 200 cm3 de tétrahydrofuranne et 10,09 g de 0,0-diéthylterbutyloxycarbonylméthyl phosphonate, agite pendant 16 heures à -15°C, verse le mélange réactionnel dans l'eau, extrait à l'éther éthylique, concentre à sec par distillation sous pression réduite, chromatographie le résidu sur silice en éluant par un mélange d'hexane et d'acétate d'éthyle (8/2) et obtient 13,48 g de produit attendu.

Spectre IR (chloroforme):

| | |
|---|---|
| 1 700 $cm^{-1}$ | C=O |
| 1 634 $cm^{-1}$ | C = C |
| 1 602 $cm^{-1}$ | aromatique |
| 1 590 $cm^{-1}$ | |
| 1 506 $cm^{-1}$ | |
| 1 487 $cm^{-1}$ | |
| 982 $cm^{-1}$ | -CH = CH- |

Spectre de RMN (CD $Cl_3$)

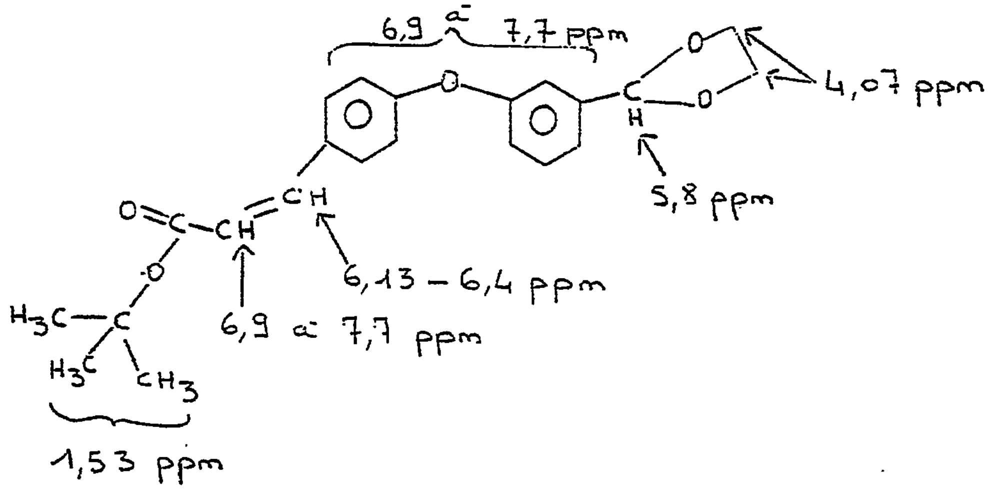


**<u>Stade C</u>** mélange de : diméthoxy méthyl 3-(4-/delta E (1,1-diméthoxy éthoxy) 1-oxopropényl) phénoxy benzène et de méthoxy méthyl 3-(4-/delta E (1,1-diméthoxy éthoxy) 1-oxopropényl/ phénoxy) benzène.

On mélange 1,5 g d'hydroxyde de palladium déposé sur sulfate de baryum à 5 % de palladium et 10 cm3 de méthanol, introduit une solution de 12 g de 2-/3-(4-/delta E (1,1-diméthyléthoxy 1-oxopropényl/phénoxy) phényl/ 1,3-dioxolane, agite sous hydrogène pendant 1 heure et 15 minutes en absorbant 717 cm3 d'hydrogène, on filtre, concentre à sec le filtrat par distillation sous pression réduite, chromatographie le résidu sur silice en éluant par un mélange d'hexane et d'acétate d'éthyle (9/1) et isole 7,16 g d'un mélange 50/50 des deux composés :

Spectre IR (chloroforme):

| | |
|---|---|
| 1 605 $cm^{-1}$ | aromatique |
| 1 586 $cm^{-1}$ | |
| 1 506 $cm^{-1}$ | |
| 1 483 $cm^{-1}$ | |
| 1 250 $cm^{-1}$ | 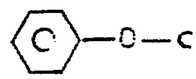 |
| 1 720 $cm^{-1}$ | C=O de $CO_2$ tBu |
| 1 369 $cm^{-1}$ | méthyle |
| 1 147 $cm^{-1}$ | C-O-C |

Spectre de RMN (CD $Cl_3$)

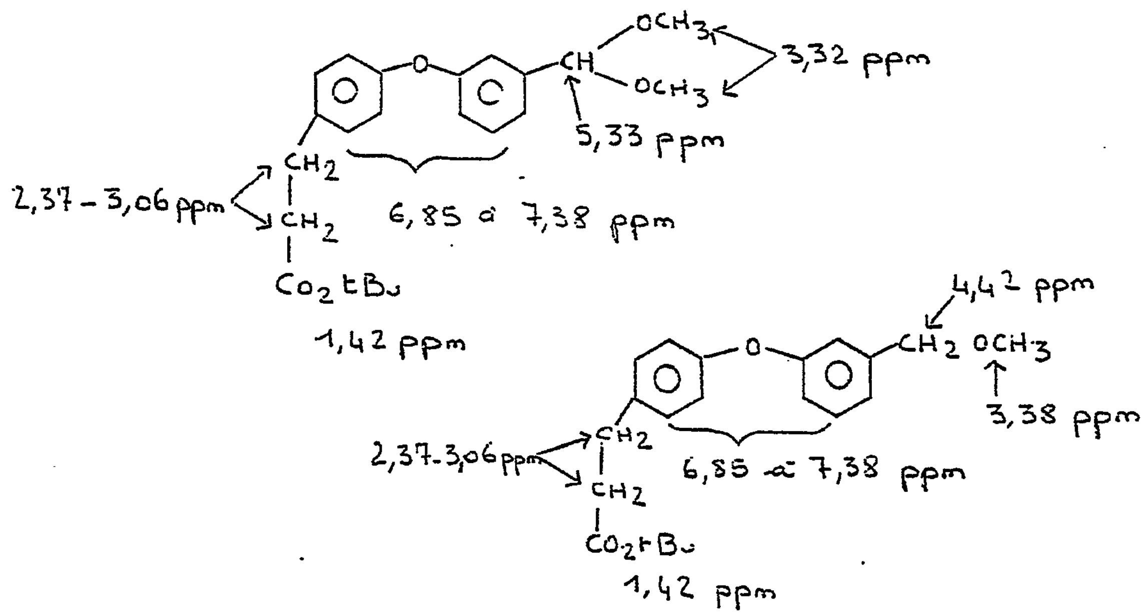


<u>**Stade D**</u> : 3/4/(1,1-diméthyléthoxy) 1-oxopropyl/phénoxy/benzaldéhyde.

On mélange 6 g de mélange de composés obtenu au stade C, 30 cm3 d'acétone et 24 cm3 de solution aqueuse N d'acide chlorhydrique, ajoute une quantité d'acétone nécessaire pour obtenir une seule phase (soit 42 cm3), agite pendant 2 heures et 30 minutes à 20°C, verse dans l'eau, extrait au chlorure de méthylène, concentre à sec par distillation sous pression réduite, chromatographie le résidu sur silice en éluant par un mélange d'hexane et d'acétate d'éthyle (9/1) et obtient 2,74 g d'aldéhyde attendu.

Spectre IR (chloroforme):

| | |
|---|---|
| 2 740 $cm^{-1}$<br>2 820 $cm^{-1}$ | C-H |
| 1 701 $cm^{-1}$ | C = 0 |
| 1 720 $cm^{-1}$ | C = 0 de $CO_2$ tBu |

Spectre de RMN :

1,45 ppm H du ter butyle
2,38 à 3,1 ppm H de $CH_2$
6,9 à 7,65 ppm H aromatiques
10,01 ppm H de CHO.

<u>**Stade E**</u> : /1R-(1 alpha (S), 3 alpha) /3-(2,2-dibromoéthényl) 2,2-diméthyl cyclopropane carboxylate de cyano/3-/4-/3-(1,1-diméthyl éthoxy) 3-oxopropyl/ phénoxy/ phényl/ méthyle et /1R-(1 alpha (R), 3 alpha/ 3-(2,2-dibromoéthényl) 2,2-diméthyl cyclopropane carboxylate de cyano/ 3-/4-/3-(1,1-diméthyléthoxy) 3-oxopropyl/phénoxy/phényl/méthyle.

On mélange 0,45 g de cyanure de sodium 0,0137 g de bromure de tétradécyl triméthyl ammonium, 8,22 cm3 d'eau, introduit à 20°C sous agitation vigoureuse un mélange de 2,74 g de 3-/4-/(1,1-diméthyléthoxy) 1-oxopropyl/ phénoxy/ benzaldéhyde obtenu au stade D, 0,0137 g de bromure de tétradécyl triméthyl ammonium, 2,74 cm3 de toluène et un mélange de 2,68 g de chlorure de l'acide 1R, cis 3-(2,2-dibrom-éthényl) 2,2-diméthyl cyclopropane carboxylique et de 2,74 cm3 de toluène, agite pendant 3 heures à 20°C, ajoute une solution aqueuse N d'acide chlorhydrique, jusqu'à pH = 7, décante, lave à l'eau la phase organique, extrait au toluène la phase aqueuse, concentre à sec les solutions toluéniques réunies par distillation sous pression réduite, chromatographie le résidu sur silice en éluant par un mélange d'hexane et d'acétate d'éthyle (9/1) et obtient 4,92 gde mélange brut des isomères (R) et (S).

On chromatographie le mélange brut sur silice en éluant par un mélange d'hexane et d'éther isopropylique (7/3) et obtient 3 fractions.

a/. 1,46 g de fraction contenant l'isomère R,
b/. 2,64 g de fraction contenant un mélange des isomères R et S.
c/. 0,395 g de fraction contenant l'isomère S.

La fraction b) est à son tour purifiée par chromatographie sur silice en éluant par un mélange d'hexane et d'éther isopropylique (7/3) et obtient 2 fractions :

d/ 0,67 g de fraction contenant l'isomère R
e/ 1,66 g de fraction contenant l'isomère S

Spectre IR (chloroforme):

| (chloroforme) | isomère R | isomère S |
|---|---|---|
| 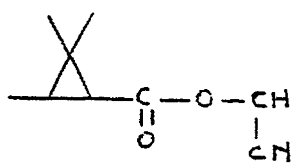 | 1 736 $cm^{-1}$ | 1 736 $cm^{-1}$ |
| 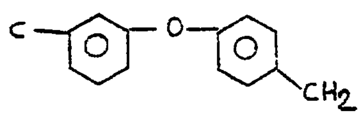 | 1 603 $cm^{-1}$<br>1 591 $cm^{-1}$<br>1 506 $cm^{-1}$ | 1 603 $cm^{-1}$<br>1 591 $cm^{-1}$<br>1 506 $cm^{-1}$ |
| | 1 487 $cm^{-1}$ (max) | 1 487 $cm^{-1}$ (max) |
| 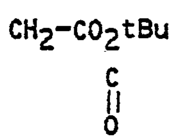 | 1 722 $cm^{-1}$ | 1 722 $cm^{-1}$ |
| Me | 1 370 $cm^{-1}$ | 1 370 $cm^{-1}$ |
| C-O-C | 1 148 $cm^{-1}$ | 1 148 $cm^{-1}$ |

Spectre de RMN (CD $Cl_3$)

| | isomère R | isomère S |
|---|---|---|
| hydrogènes des méthyles géminés | 1,31 ppm | 1,2-1,25 ppm |
| hydrogène de trBu | 1,44 ppm | 1,42 ppm |
| les hydrogènes du cyclopropyle | 1,82 à 2,18 ppm | 1,75 à 2,2 ppm |
| les hydrogènes des $CH_2$ de la chaîne alkyle | 2,37 à 3,07 ppm | 2,33 à 3,0 ppm |
| hydrogène de -CH-CN | 6,33 ppm | 6,37 ppm |
| hydrogènes éthyléniques | 6,65 à 6,7 ppm | 6,63 à 6,77 ppm |
| hydrogènes aromatiques | 6,88 à 7,43 ppm | 6,86 à 7,4 ppm |

Dichroïsme circulaire:

| isomère R | isomère S |
|---|---|
| Max $\leqslant$ 230 nm $\Delta\varepsilon = -9$ | max vers 225 mn $\Delta\varepsilon = +11$ |
| Max 268 nm $\Delta\varepsilon = -0,4$ | max 285 mn |
| Max 280 nm $\Delta\varepsilon = -0,45$ | $\Delta\varepsilon = + 0,4$ |

**Stade F :** Acide 1R-/1 alpha (R), 2 alpha/ 4-/3-/cyano/3-(2,2-dibromoéthényl) 2,2-diméthyl cyclopropyl/ carbonyl/oxy/méthyl/phénoxy/benzène propanoïque.

On mélange 1,1 g d'ester terbutylique de l'isomère R obtenu au stade précédent avec 55 cm3 de toluène, porte au reflux, ajoute 0,1 g d'acide paratoluène sulfonique, maintient le reflux pendant 30 minutes, refroidit, filtre, concentre à sec par distillation sous pression réduite, chromatographie le résidu sur silice en éluant par un mélange d'hexane et d'acétate d'éthyle (1/1) et obtient 0,93 g d'acide attendu.

Spectre IR (chloroforme):

1 742 $cm^{-1}$ C=O ester

1 604 $cm^{-1}$
1 591 $cm^{-1}$
1 509 $cm^{-1}$
1 487 $cm^{-1}$ } aromatiques

1 712 $cm^{-1}$ acide C=O dimère

Spectre RMN (CD $Cl_3$)

| | |
|---|---|
| hydrogènes des méthyles géminés et du tBu | 1,3 ppm |
| hydrogènes du cyclopropyle (cis) | 1,8 à 2,19 ppm |
| les hydrogènes des $CH_2$ de la chaîne alkyle | 2,5 à 3,17 ppm |

| | |
|---|---|
| hydrogène de -CH-CN | 6,23 ppm |
| hydrogène éthylénique | 6,6-6,73 ppm |
| hydrogènes aromatiques | 6,88 à 7,43 ppm |

**Exemple 2 : acide 1R-/1 alpha (S), 2 alpha /4-/3-/cyano///3-(2,2-dibromo éthényl) 2,2-diméthyl cyclopropyl/carbonyl/ oxy/ méthyl/ phénoxy/ benzène propanoïque.**

On mélange 1,805 g d'ester terbutylique (S) obtenu à l'exemple 1 avec 90,25 cm3 de toluène, porte au reflux, ajoute 0,18 g d'acide paratoluène sulfonique, maintient le reflux pendant 30 minutes, refroidit, filtre, concentre à sec par distillation sous pression réduite, chromatographie le résidu sur silice en éluant par un mélange d'hexane et d'acétate d'éthyle (1/1) et obtient 1,33 g d'acide attendu.

Spectre IR (chloroforme):

| | |
|---|---|
| 1 742 $cm^{-1}$ | $C=O$ ester |
| 1 604 $cm^{-1}$ | aromatiques |
| 1 591 $cm^{-1}$ | |
| 1 509 $cm^{-1}$ | |
| 1 487 $cm^{-1}$ | |
| 1 712 $cm^{-1}$ | acide $C=O$ dimère |

Spectre RMN (CO $Cl_3$)

| | |
|---|---|
| les hydrogènes des méthyles géminés : | 1,2-1,25 ppm |
| les hydrogènes du cyclopropyle: | 1,82 à 2,23 ppm |
| les hydrogènes des $CH_2$ de la chaîne alkyle | 2,5 à 3,17 ppm |
| hydrogène de -CH-CN | 6,43 ppm |
| hydrogène éthylénique | 6,68 à 7,16 ppm |
| hydrogènes aromatiques | 6,97 à 7,7 ppm |

**Exemple 3 : /1R /1 alpha (S), 2 alpha /4-/3-/cyano///3-(2,2-dibromo éthényl) 2,2-diméthyl cyclopropyl/ carbonyl/ oxy/ méthyl/ phénoxy/ benzène N-/2-(2-iodo$^{125}$I 4-1H imidazolyl) éthyl/propanamide.**

**Stade A** : Préparation de l'anhydride mixte en solution (solution A).

On mélange sous argon 25 µl de dioxane anhydre, 10 µl de tri-n-butylamine dilué au 1/125 dans du dioxane anhydre et 10 µl de chloroformiate d'isobutyle dilué au 1/250 dans du dioxane anhydre, ajoute une solution de 150 g d'acide 1R /1 alpha (S), 2 alpha/ 4-/3-/cyano///3-(2,2-dibromoéthényl) 3,3-diméthyl cyclopropyl/ carbonyl/oxy/méthyl/phénoxy/benzène propanoïque en solution dans 25 µl de dioxane anhydre, maintient 30 minutes à 20°C sous agitation, dilue le mélange réactionnel par 125 µl de dioxane anhydre et obtient la solution A d'anhydride mixte.

**Stade B** : Marquage de l'histamine

On mélange à 20°C 10 µl de solution d'histamine base 2 mM dans du tampon au phosphate de sodium 0,5 M à pH 7,4, 1 m Ci de $^{125}$ INa, 10 µl de solution aqueuse de chloramine T à 5 mg/ml, après 90 secondes, on ajoute 10 µl de solution aqueuse de métabisulfite de sodium à 25 mg/ml.

Le milieu obtenu B est contrôlé par chromatographie. (voir tableau A).

**Stade C** : Condensation :

Dans le tube contenant le milieu réactionnel B, on introduit 50 l de solution A d'anhydride mixte, conserve pendant 2 heures à 4°C. On obtient alors la solution C qui est analysée par chromatographie (voir tableau B).

**Stade D** : Purification.

Le milieu réactionnel C est purifié par HPLC sur une colonne Zorbax Golden Sil 100 × 7,5 nm, 3 m, élué par le mélange chloroforme-méthanol (92/8). La radioactivité de l'effluent est suivie grâce à un détecteur à cristal d'iodure de sodium Beckman 170 couplé à un enregistreur intégrateur. On obtient 4 pics (voir tableau I). Seul le pic 3 contient un antigène qui se lie à un anticorps "antideltaméthrine". Les fractions contenant le produit immunoréactif sont réunies. La solution D (tableau C) obtenue est concentrée à sec sous argon, le résidu est additionné de 5 ml d'éthanol. On obtient environ 600 µCi de conjugué radioactif. Le produit stocké à -30° C est stable pendant au moins 2 mois.

**Exemple 4 : /1R /1 alpha (R), 2 alpha /4-/3-/cyano///3-(2,2-dibromo éthényl) 2,2-diméthyl cyclopropyl/ carbonyl/oxy/méthyl/phénoxy/benzène N-/2-(2-iodo$^{125}$I 4-1H-imidazolyl) éthyl/propanamide.**

**Stade A** : Préparation de l'anhydride mixte en solution (solution A).

On mélange, sous argon, 25 µl de dioxane anhydre, 10 µl de tri-n-butyl amine dilué au 1/125 dans du dioxane anhydre, 10 µl de chloroformiate d'isobutyle dilué au 1/250 dans du dioxane anhydre, ajoute 150 µg d'acide 1 R /1 alpha (R), 2 alpha/ 4-/3-/cyano///3-(2,2-dibromoéthényl) 2,2-diméthyl cyclopropyl/ carbonyl/ oxy/ méthyl/phénoxy/benzène propanoïque en solution dans 25 µl de dioxane anhydre, conserve pendant 30 minutes à 20°C, dilue le mélange réactionnel par 125 µl de dioxane anhydre et obtient la solution A d'anhydride mixte.

**Stade B** : Marquage de l'histamine

On mélange à 20°C 10 µl de solution d'histamine base 2 mM dans du tampon phosphate de sodium 0,5 M à pH 7,4, 1 m Ci de $^{125}$I Na, 10 µl de solution aqueuse de chloramine T à 5 mg/ml, après 90 secondes, on ajoute 10 µl de solution aqueuse de métabisulfite de sodium à 25 mg/ml.

Le milieu obtenu B est contrôlé par chromatographie (voir tableau A).

**Stade C** : Condensation

Dans le tube contenant le milieu réactionnel B, on introduit 50 l de solution A d'anhydride mixte, conserve pendant 2 heures à 4°C. On obtient alors la solution C qui est analysée par chromatographie (voir tableau B).

**Stade D** : Purification.

Le milieu réactionnel C est purifié par HPLC sur une colonne Zorbax Golden Sil 100 × 7,5 nm, 3 µm, éluée par le mélange chloroforme-méthanol (97/3). La radioactivité de l'effluent est suivie grâce à un détecteur à cristal d'iodure de sodium Berthold. On obtient 4 pics (voir tableau I). Seul le pic 3 contient un antigène capable de se lier aux anticorps "antideltaméthrine". Les fractions contenant le produit immunoréactif sont réunies. La solution D (tableau C) obtenue est évaporée à sec sous argon. Le résidu est additionné de 5 ml d'éthanol. On obtient 220 µCi de conjugué radioactif. Le produit stocké à -30° C est stable pendant au moins 2 mois.

## Revendications

1. Les produits de formule générale (I):

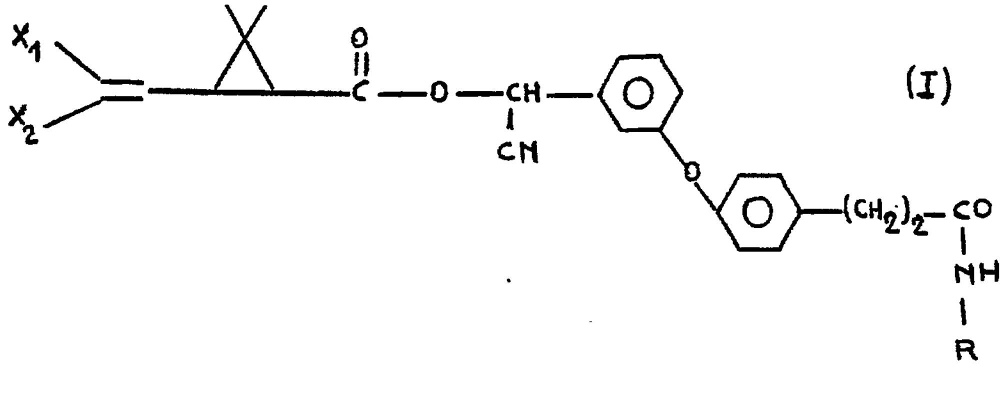

(I)

dans laquelle $X_1$ représente un atome d'halogène ou un radical trifluorométhyle, $X_2$ représente un atome

d'halogène et R représente le reste d'un acide aminé R–$NH_2$ ou le reste d'un dérivé de ce dernier choisi dans le groupe constitué par l'histidine, la tyrosine, l'histidinol, l'histamine, la tyramine et le tyrosinate de méthyle marqués à l'iode $^{125}$ ou $^{131}$, ces produits se présentant sous l'une quelconque de leurs formes isomères ou sous forme de mélanges d'isomères.

2. Les produits de formule générale (I) telle que définie à la revendication 1 dans laquelle $X_1$ représente un atome de chlore ou de brome ou un radical trifluorométhyle et $X_2$ représente un atome de chlore ou de brome.

3. Les produits de formule générale (I) telle que définie à la revendication 1 ou 2 dans laquelle $X_1$ et $X_2$ représente un atome de brome.

4. 1R-[1 alpha S, 2 alpha] 4-[3-[cyano[[[3-(2,2 dibromoéthényl)-2,2-diméthyl cyclopropyl]carbonyl]-oxy]méthyl]phenoxy]benzène-N-[2-(2-iodo $^{125}$I 4-1H-imidazolyl)-éthyl]propanamide de formule

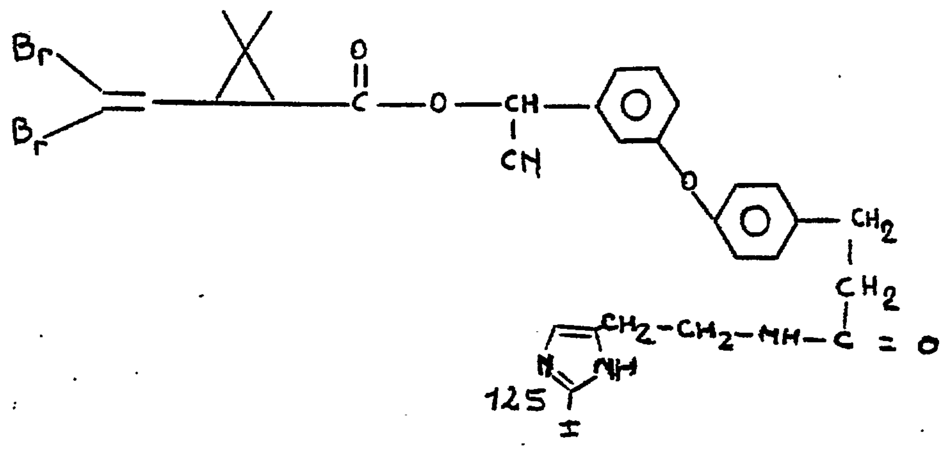

5. Procédé de préparation des produits de formule (I) telle que définie à la revendication 1, caractérisé en ce que l'on fait réagir un produit de formule:

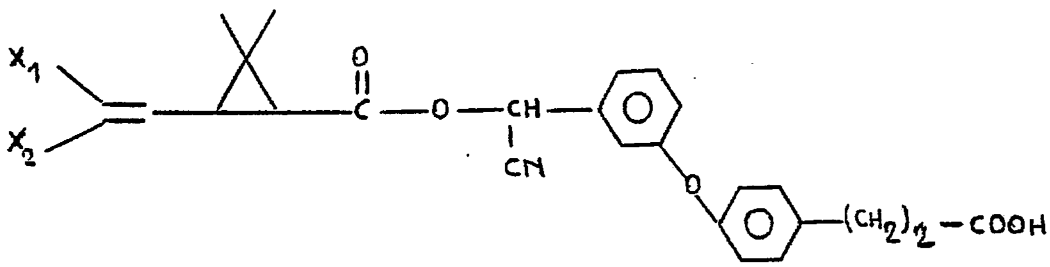

dans laquelle $X_1$ et $X_2$ ont les significations indiquées à la revendication 1, ce produit se présentant sous l'une quelconque de ses formes isomères ou sous forme de mélanges d'isomères, avec un halogénoformiate d'alcoyle dans lequel le radical d'alcoyle $R_1$ renferme au plus 6 atomes de carbone pour obtenir le produit de formule (III):

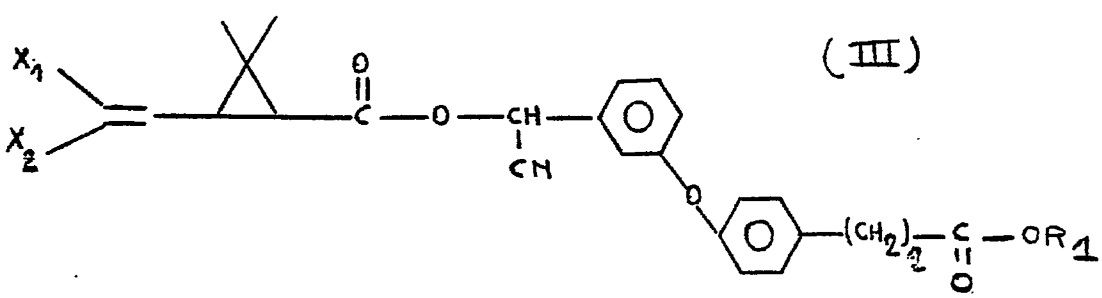

(III)

dans laquelle $X_1$ et $X_2$ ont les spécifications précitées que l'on fait réagir avec un acide aminé ou un dérivé d'acide aminé choisi dans le groupe constitué par l'hystidine, la tyrosine, l'histidinol, l'histamine, la tyramine et tyrosinate de méthyle marqués à l'iode $^{125}$ ou $^{131}$ et obtient le produit recherché de formule générale (I) que l'on purifie par des moyens physiques.

6. Procédé selon la revendication 5 ou 6 caractérisé en ce que la réaction avec l'halogénoformiate d'alcoyle est effectuée en présence d'une base tertiaire, en milieu anhydre et sous atmosphère inerte.

7. Procédé selon la revendication 6 caractérisé en ce que l'halogénoformiate d'alcoyle est le chloroformiate d'isobutyle en présence de tri-n-butylamine.

8. Procédé suivant l'une quelconque des revendications 5 à 7 caractérisé en ce que l'acide aminé ou son dérivé que l'on fait réagir avec le produit de formule (III) dans laquelle $R_1$ représente un groupe activateur de la fonction carbonyle, est l'histamine marquée à l'iode $^{125}$ ou $^{131}$ ou un dérivé et on opére sous atmosphère inerte.

9. Procédé suivant l'une quelconque des revendications 5 à 8 caractérisé en ce que l'on obtient le produit de formule:

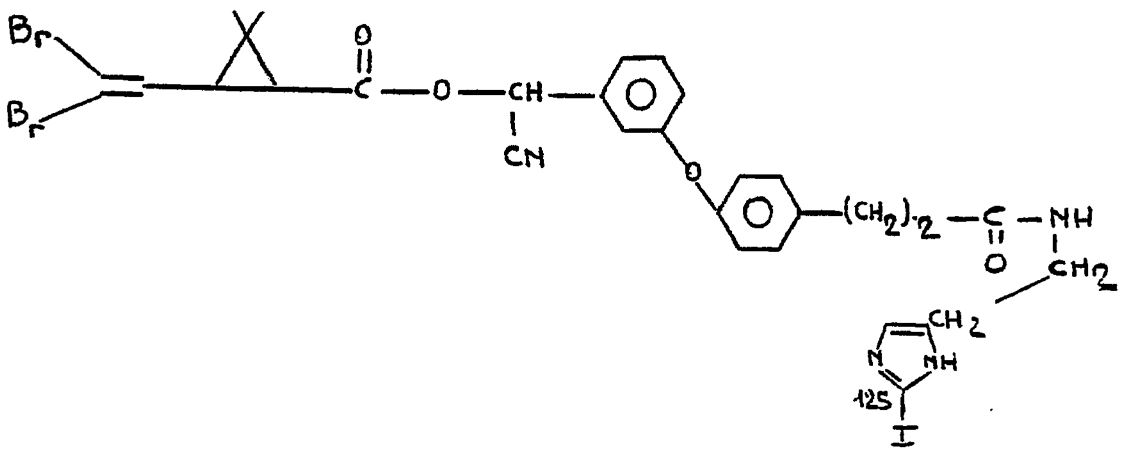

10. Application des produits de formule générale (I) telle que définie dans la revendication 1, à l'étude et au dosage radioimmunologique des esters de l'alcool alpha-cyano 3-phénoxy benzylique correspondants dans les fluides biologiques chez l'homme ou l'animal.

11. Application selon la revendication 10 du 1R-[1 alpha S, 2 alpha] 4-[3-[cyano [[[3-(2,2 dibromoéthényl)-2,2-diméthyl cyclopropyl]carbonyl]oxy]méthyl]phenoxy]benzène-N-[2-(2-iodo $^{125}$I 4-1H-imidazolyl)-éthyl]-propanamide à l'étude et au dosage radioimmunologiques de la deltaméthrine dans les fluides biologiques chez l'homme ou l'animal.

**Claims**

1. The products of general formula (I):

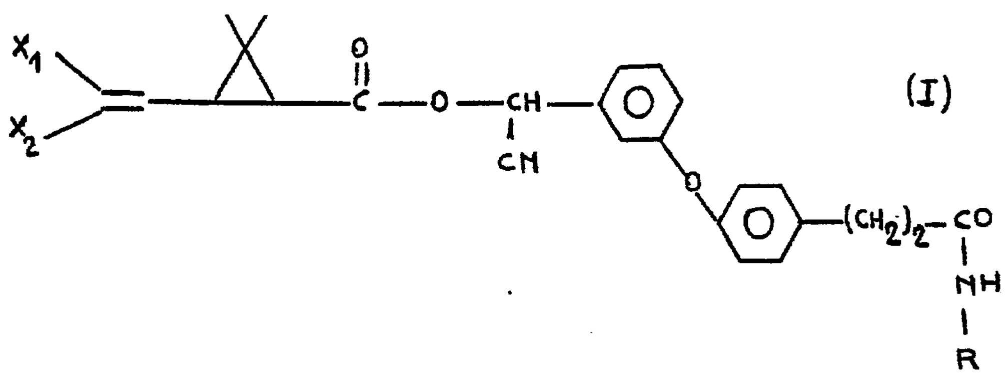

(I)

in which $X_1$ represents a halogen atom or a trifluoromethyl radical, $X_2$ represents a halogen atom and R represents the residue of an $R{-}NH_2$ amino acid or the residue of a derivative of this latter chosen from the group constituted by histidine, tyrosine, histidinol, histamine, tyramine and methyl tyrosinate labelled with iodine $^{125}$ or $^{131}$, these products being presented in any of their isomer forms or in the form of isomer mixtures.

2. The products of general formula (I) as defined in claim 1 in which $X_1$ represents a chlorine or bromine atom or a trifluoromethyl radical and $X_2$ represents a chlorine or bromine atom.

3. The products of general formula (I) as defined in claim 1 or 2 in which $X_1$ and $X_2$ represents a bromine atom.

4. 1R-[1-alpha S, 2-alpha]-4-[3-[cyano-[[[3-(2,2-dibromoethenyl)-2,2-dimethylcyclopropyl]carbonyl]-oxy]methyl]phenoxy]benzene-N-[2-(2-iodo $^{125}I$ 4-1H-imidazolyl)-ethyl]-propanamide of formula

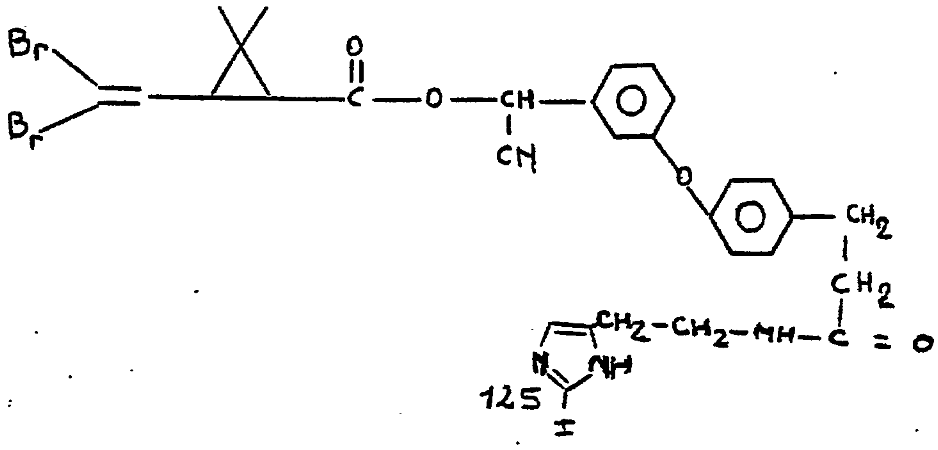

5. Process for the preparation of products of formula (I) as defined in claim 1, characterized in that a product of formula:

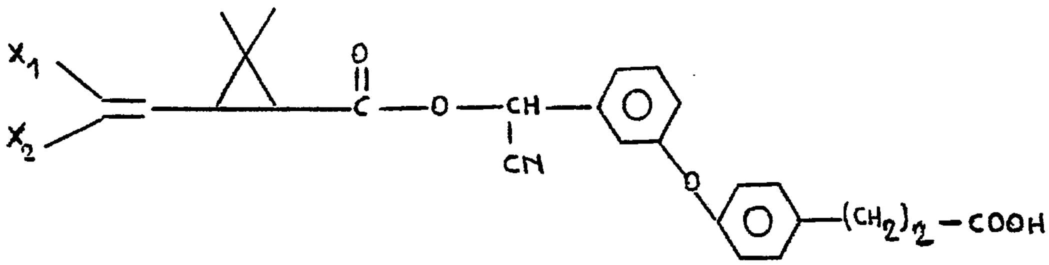

in which $X_1$ and $X_2$ have the meanings indicated in claim 1, this product being presented in any of its isomer forms or in the form of isomer mixtures, is reacted with an alkyl halogenoformate in which the alkyl radical $R_1$ contains at most 6 carbon atoms, so as to obtain the product of formula (III):

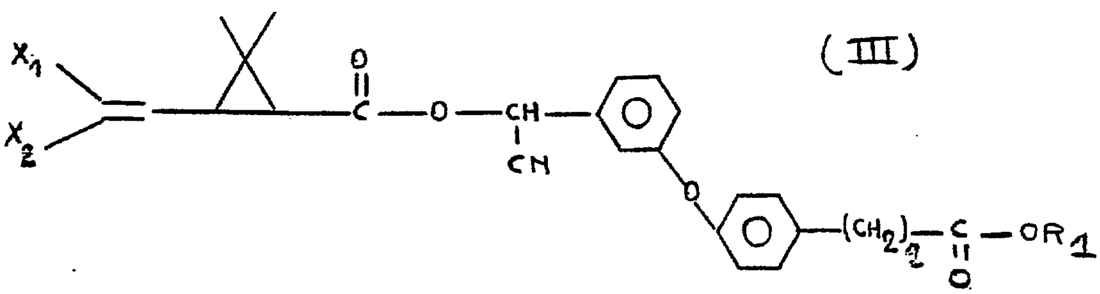

in which $X_1$ and $X_2$ have the previously-mentioned specifications, which is reacted with an amino acid or an amino acid derivative chosen from the group constituted by histidine, tyrosine, histidinol, histamine, tyramine and methyl tyrosinate labelled with iodine $^{125}$ or $^{131}$ and the desired product of general formula (I) is obtained, which is purified by physical means.

6. Process according to claim 5 or 6 characterized in that the reaction with alkyl halogenoformate is carried out in the presence of a tertiary base, in an anhydrous medium and under inert atmosphere.

7. Process according to claim 6, characterized in that the alkyl halogenoformate is isobutyl chloroformate in the presence of tri-n-butylamine.

8. Process according to any one of claims 5 to 7, characterized in that the amino acid or its derivative that is reacted with the product of formula (III), in which $R_1$ represents an activating group for the carbonyl function, is histamine labelled with iodine $^{125}$ or $^{131}$ or a derivative and the operation is carried out under inert atmosphere.

9. Process according to any one of claims 5 to 8, characterized in that the product of formula:

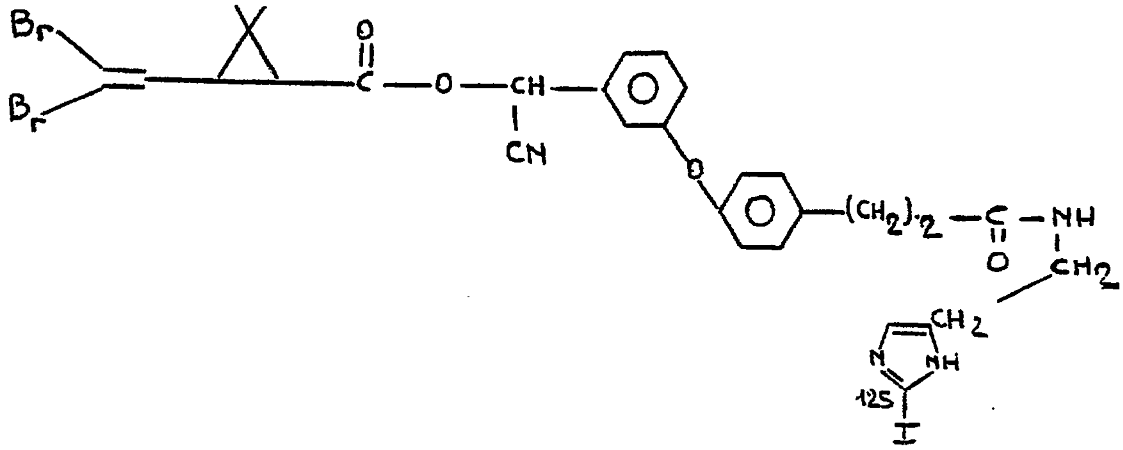

is obtained.

10. Use of the products of general formula (I) as defined in claim 1, in radioimmunological study and determination of corresponding esters of alpha-cyano-3-phenoxy benzyl alcohol in biological fluids in man or in animals.

11. Use according to claim 10 of 1R-[1-alpha S, 2-alpha]-4[3-[cyano-[[[3-(2,2-dibromoethenyl)-2,2-dimethylcyclopropyl]carbonyl]oxy]methyl]phenoxy]-benzene-N-[2-(2-iode $^{125}$I 4-1H-imidazolyl)-ethyl]-propanamide in radioimmunological study and determination of deltamethrine in biological fluids in man or in animals.

**Patentansprüche**

1. Produkte der allgemeinen Formel (I)

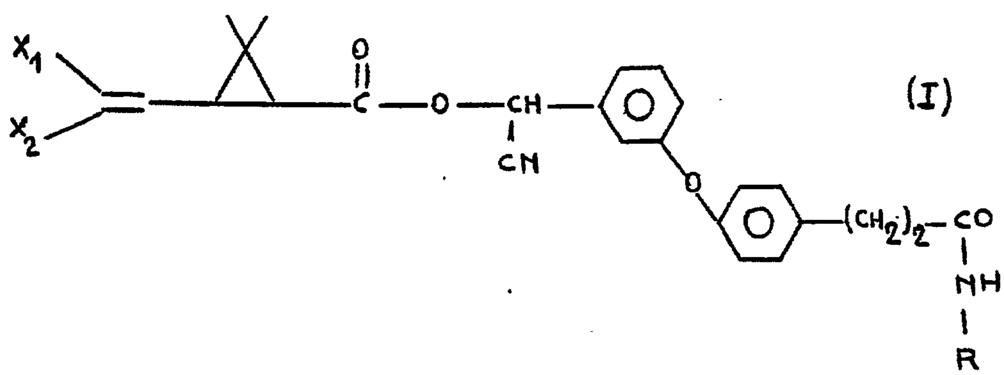

(I)

worin $X_1$ für ein Halogenatom oder eine Trifluormethylgruppe steht, $X_2$ für ein Halogenatom steht und R den Rest einer Aminosäure $R{-}NH_2$ oder den Rest eines Derivats dieser letzteren, ausgewählt aus Histidin, Tyrosin, Histidinol, Histamin, Tyramin und Methyltyrosinat, markiert mit Jod$^{125}$ oder $^{131}$, bedeutet, wobei diese Produkte in irgendeiner ihrer isomeren Formen oder in Form eines Gemisches der Isomeren vorliegen.

2. Produkte der allgemeinen Formel (I) gemäß Anspruch 1, worin $X_1$ für ein Chlor- oder Bromatom oder eine Trifluormethylgruppe steht und $X_2$ ein Chlor- oder Bromatom wiedergibt.

3. Produkte der allgemeinen Formel (I) gemäß Anspruch 1 oder 2, worin $X_1$ und $X_2$ ein Bromatom bedeuten.

4. 1R-[1αS,2α]-4-[3-[Cyano-[[[3-(2,2-dibromethenyl)-2,2-dimethyl-cyclopropyl]-carbonyl]-oxy]-methyl]phenoxy]-benzol-N-[2-(2-jod $^{125}$I-4-1H-imidazolyl)-ethyl]-propanamid der Formel

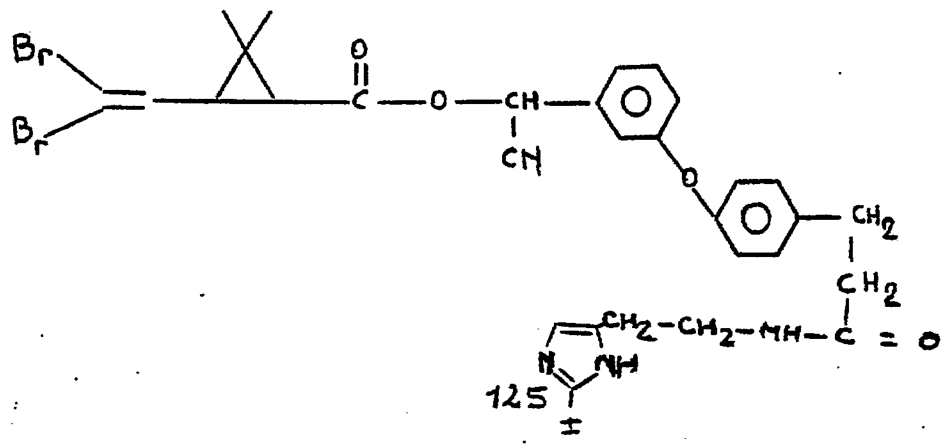

5. Verfahren zur Herstellung der Produkte der Formel (I) gemäß Anspruch 1, dadurch gekennzeichnet, daß man ein Produkt der Formel

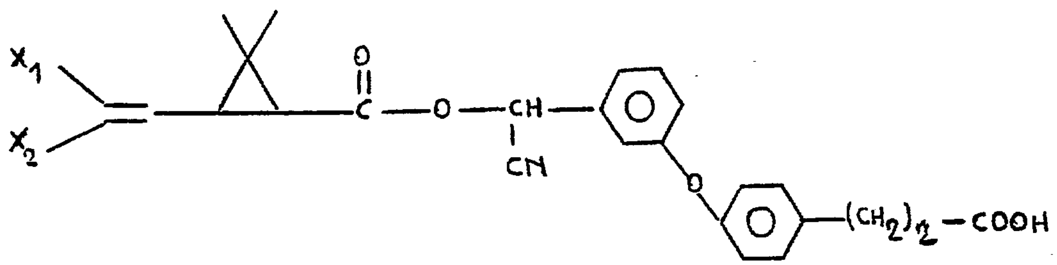

worin $X_1$ und $X_2$ die in Anspruch 1 angegebenen Definitionen besitzen, wobei dieses Produkt in irgendeiner seiner isomeren Formen oder in Form des Gemisches der Isomeren vorliegt, mit einem Alkylhalogenformiat umsetzt, worin der Alkylrest $R_1$ höchstens 6 Kohlenstoffatome enthält, um zu dem Produkt der Formel (III)

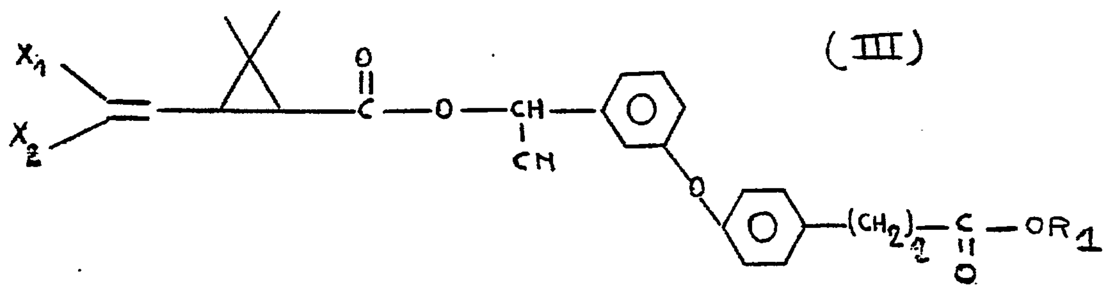

(III)

zu gelangen, worin $X_1$ und $X_2$ die vorstehend angegebenen Bedeutungen besitzen, welches man mit einer Aminosäure oder einem Aminosäurederivat, ausgewählt unter Histidin, Tyrosin, Histidinol, Histamin, Tyramin und Methyltyrosinat, markiert mit $Jod^{125}$ oder $^{131}$, umsetzt und zu dem gewünschten Produkt der allgemeinen Formel (I) gelangt, welches man mit Hilfe physikalischer Methoden reinigt.

6. Verfahren gemäß Anspruch 5, dadurch gekennzeichnet, daß die Umsetzung mit dem Alkylhalogenformiat in Gegenwart einer tertiären Base in wasserfreiem Milieu und unter inerter Atmosphäre durchgeführt wird.

7. Verfahren gemäß Anspruch 6, dadurch gekennzeichnet, daß das Alkylhalogenformiat Isobutylchlorformiat in Gegenwart von Tri-n-butylamin ist.

8. Verfahren gemäß einem der Ansprüche 5 bis 7, dadurch gekennzeichnet, daß die Aminosäure oder ihr Derivat, welches man mit dem Produkt der Formel (III) umsetzt, worin $R_1$ für eine Aktivatorgruppe der Carbonylfunktion steht, mit $Jod^{125}$ oder $^{131}$ markiertes Histamin oder ein Derivat ist und man unter inerter Atmosphäre arbeitet.

9. Verfahren gemäß einem der Ansprüche 5 bis 8, dadurch gekennzeichnet, daß man das Produkt der Formel

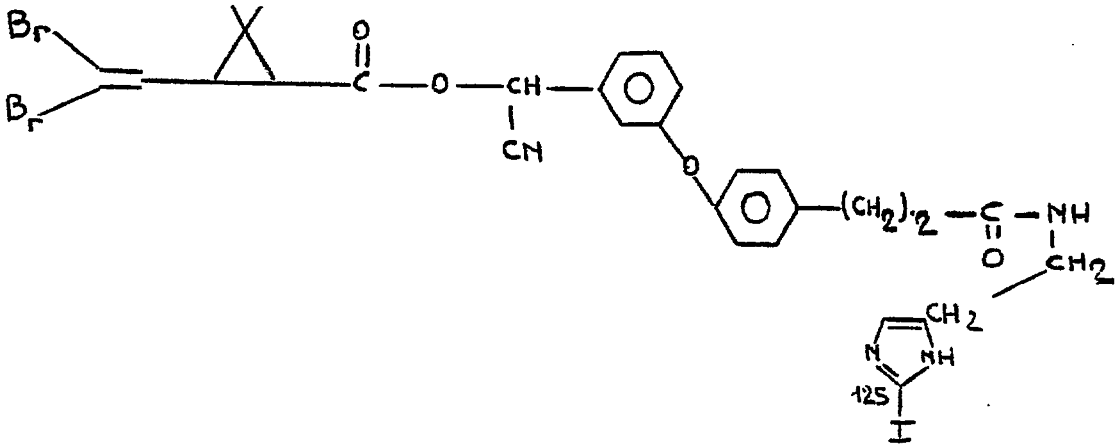

erhält.

10. Verwendung der Produkte der allgemeinen Formel (I) gemäß Anspruch 1 für die radioimmunologische Untersuchung und Bestimmung entsprechender Ester des α-Cyano-3-phenoxybenzylalkohols in biologischen Flüssigkeiten bei Mensch oder Tier.

11. Verwendung gemäß Anspruch 10 von 1R-[1αS,2α]-4-[3-[Cyano-[[[3-(2,2-dibromethenyl)-2,2-dimethyl-cyclopropyl]-carbonyl]-oxy]-methyl]-phenoxy]-benzol-N-[2-(2-jod $^{125}$I-4-1H-imidazolyl)-ethyl]-propanamid für die radioimmunologische Untersuchung und Bestimmung des Deltamethrins in biologischen Flüssigkeiten bei Mensch oder Tier.

TABLEAU 1

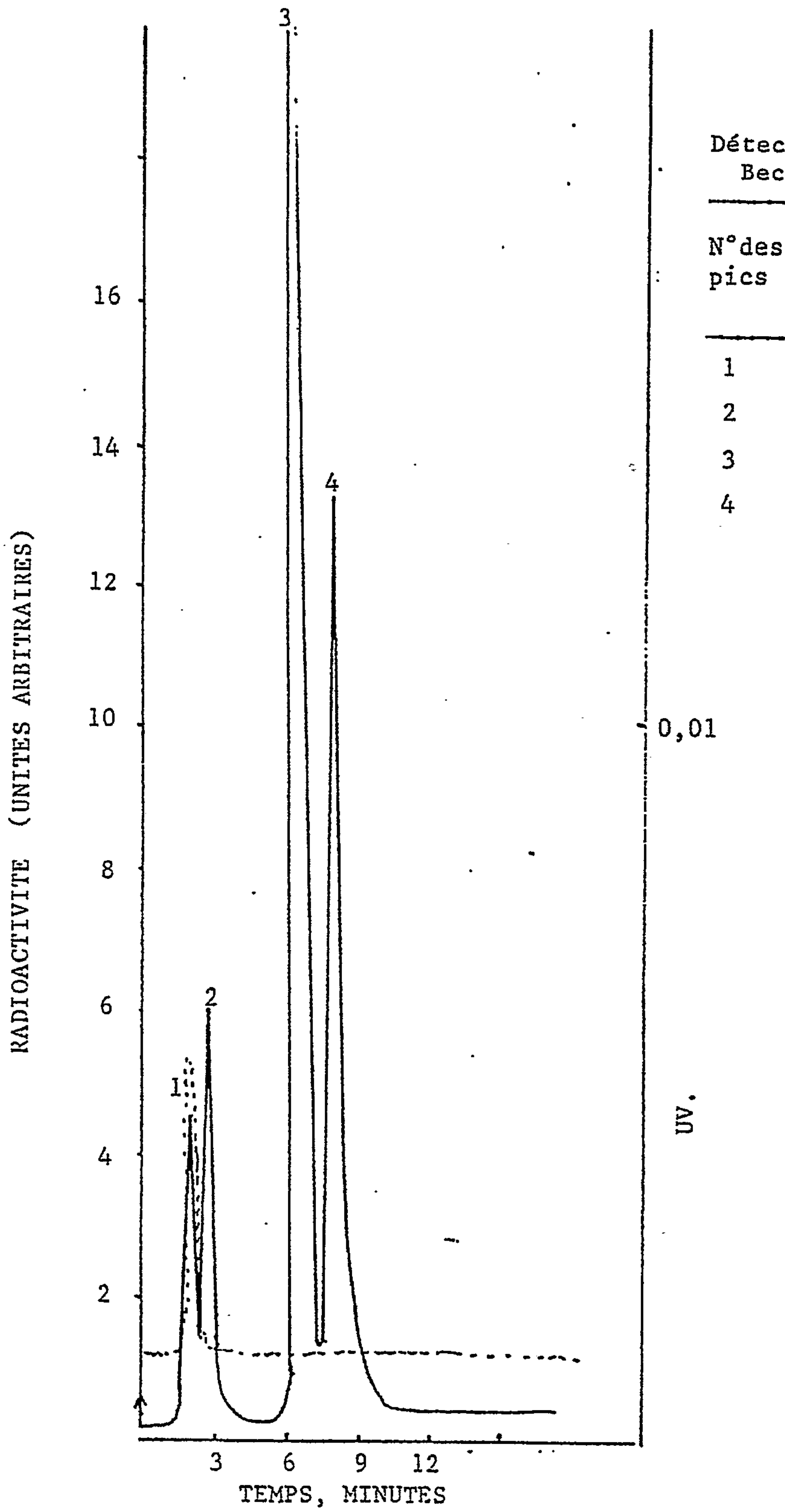


Détection de la Radioactivité
Beckman model 170

| N°des pics | tps de rétention en | % de radio-activité |
|---|---|---|
| 1 | 2,44 | 5,34 |
| 2 | 3,17 | 8,00 |
| 3 | 6,93 | 63,46 |
| 4 | 8,57 | 22,98 |

Profil HPLC du conjugué iodé. Conditions de chromatographie : colonne : Zorbax Golden Sil, 3 µm ; phase mobile : $CHCl_3$'MeOH (98/2,v/v) ; débit : 1ml/min ; Pression : 800 PSI ; Echantillon 25 µl ; détection : Radioactivité - Beckman Model 170, UV ... UVICORD LKB 276 nm 0,02.

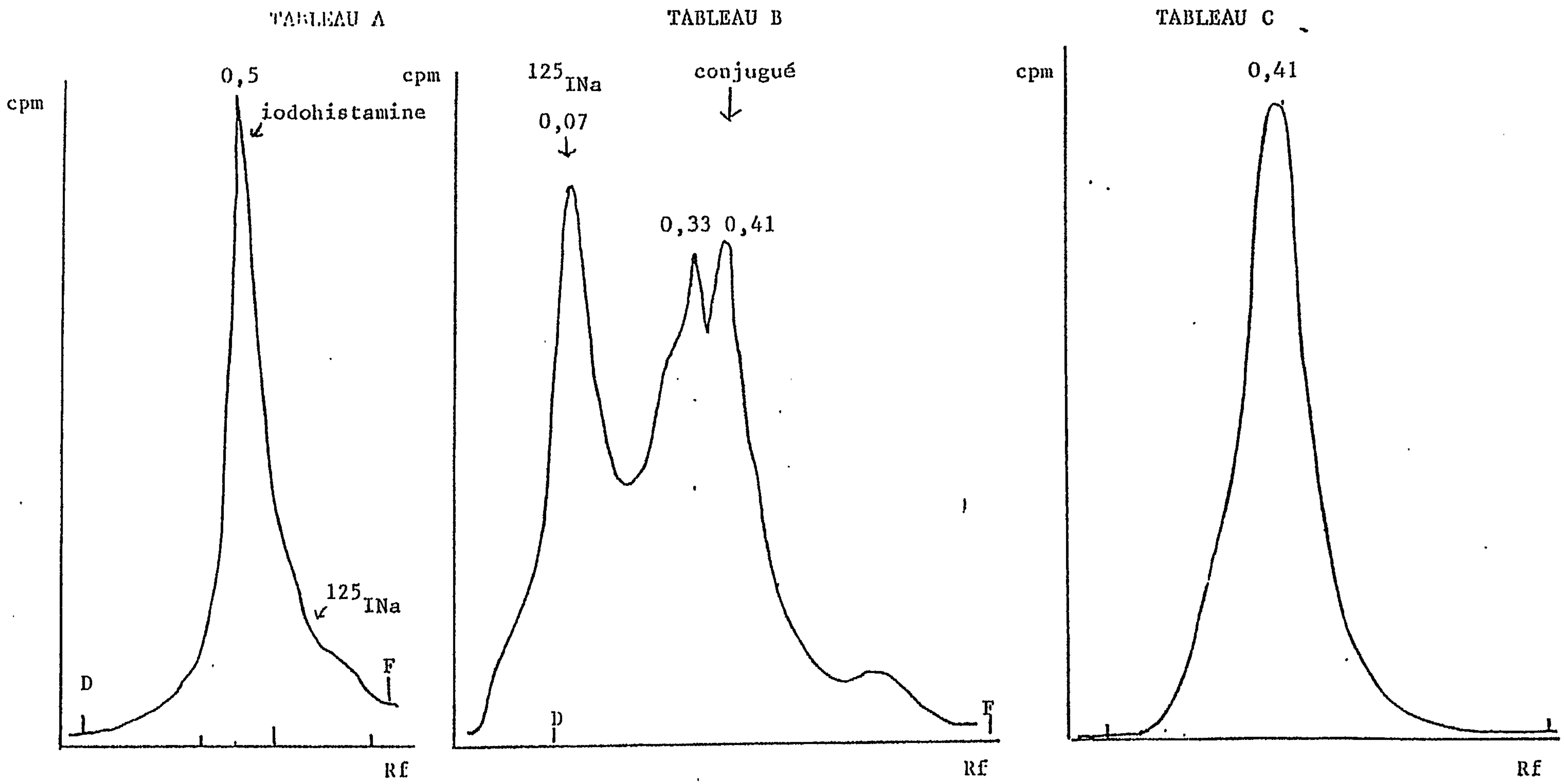


Radiochromatogrammes de A : $^{125}$Iodohistamine (milieu "B") ; B : conjugué radioactif (milieu "C") ; C pic 3 conjugué purifié (solution "D") ; Plaques de Silice Merck 60F254 ; Solvants A : EtOH-$H_2O$-$NH_4OH$ (77/18/5) ; B et C : cyclohexane - EtOH - Triéthylamine (70/30/1). Lecteur de plaques : Berthold LB 2832.

TABLEAU 1

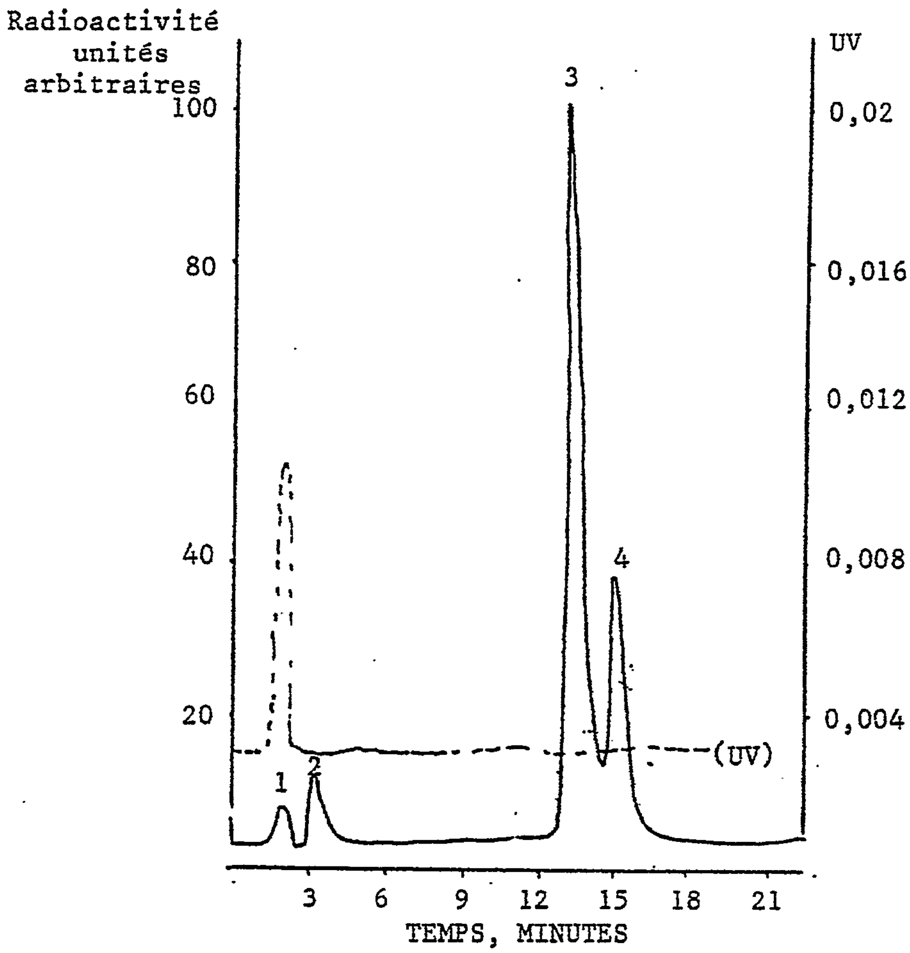


Profil HPLC du conjugué iodé - Conditions de chromatographie : Colonne : Zorbax Golden Sil, 3 um ; Phase mobile : $CHCl_3$/MeOH 97/3 (v/v) ; débit : 1 ml/min ; Pression : 800 PSI ; Echantillon 70 µl, détection : radioactivité Berthold SOOK, UV. UVICORDS LKB 276 nm 0,02.

TABLEAU A

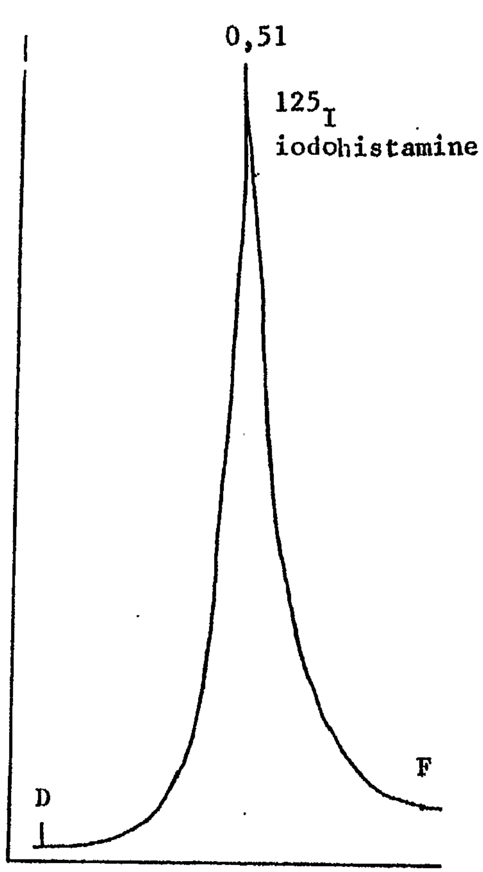


TABLEAU B

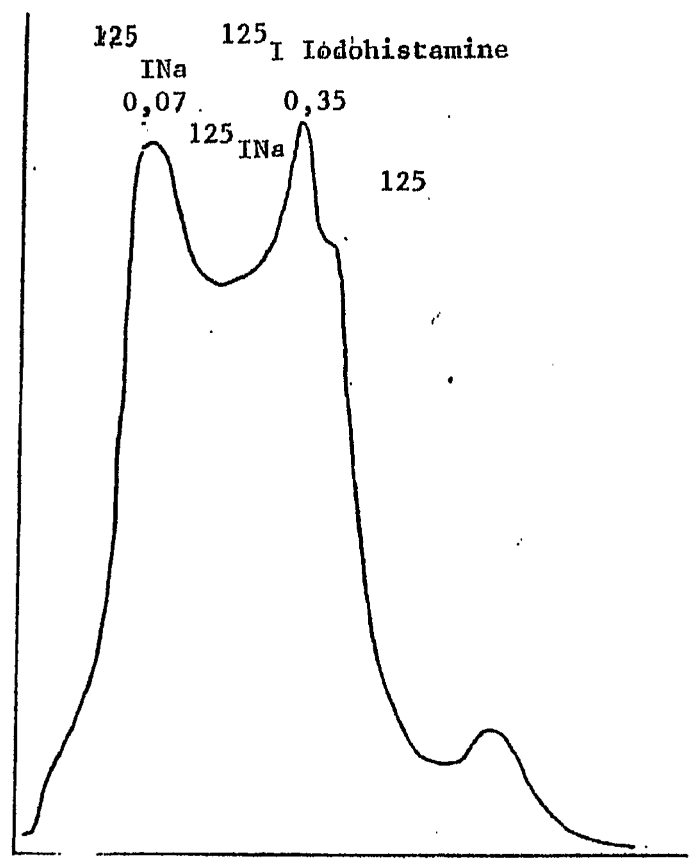


TABLEAU C

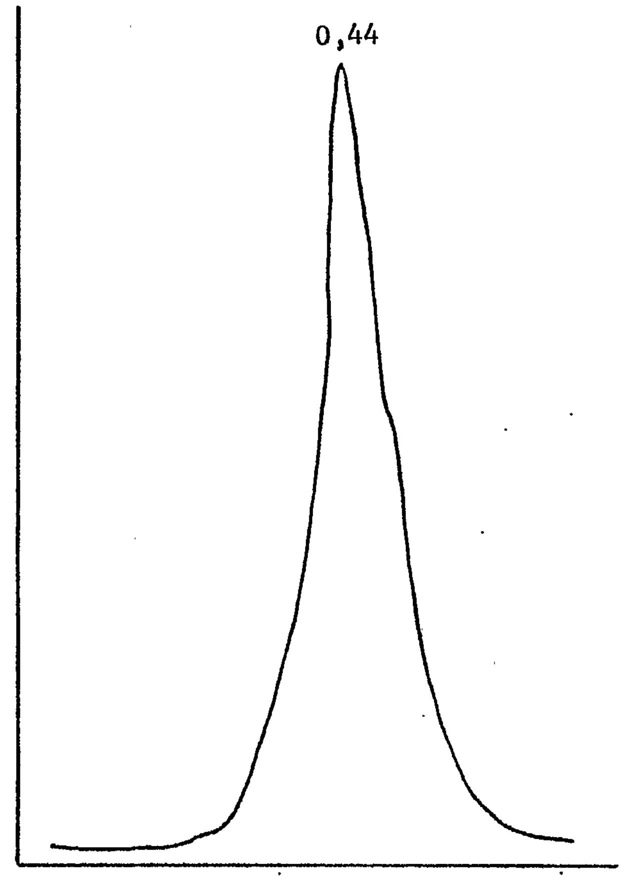


Radiochromatogrammes de A : $^{125}$Iodohistamine (milieu "B"), B : conjugué radioactif (milieu "C"), C : pic 3 (solution D), Plaques de Silice Merck 60F254, Solvants A : EtOH-$H_2O$-$NH_4OH$ (77/18/5), B et C : Cyclohexane-EtOH-Triéthylamine (70/30/1), Lecteur de plaques Berthold LB 2832.